# EUROPEAN PATENT SPECIFICATION

(11) **EP 4 640 156 B1**
(45) Date of publication and mention of the grant of the patent: **25.11.2026**
(21) Application number: 24204141.6
(22) Date of filing: 02.10.2024
(51) Int. Cl.: A61B 6/03, A61B 6/12, A61B 6/00, A61B 34/10

(54) **METHOD AND SYSTEM FOR PROVIDING PARAMETER DATA FOR A CONTROL SCAN OF A COMPUTED TOMOGRAPHY SCANNER ARRANGEMENT**
VERFAHREN UND SYSTEM ZUR BEREITSTELLUNG VON PARAMETERDATEN FÜR EINEN ÜBERPRÜFUNGSSCAN EINER COMPUTERTOMOGRAFIESCANNERANORDNUNG
PROCÉDÉ ET SYSTÈME POUR FOURNIR DES DONNÉES DE PARAMÈTRE POUR UN BALAYAGE DE VÉRIFICATION D'UN AGENCEMENT DE SCANNER DE TOMODENSITOMÉTRIE

(43) Date of publication of application: 29.10.2025
(73) Proprietor: Siemens Healthineers AG, 91301 Forchheim (DE)
(72) Inventor: BAER-BECK, Matthias, 91080 Spardorf (DE); HOFMANN, Christian, 91058 Erlangen (DE); SUNNEGAARDH, Johan, 91325 Adelsdorf (DE)
(74) Representative: Siemens Healthineers Patent Attorneys

(56) References cited:
- WO-A1-2015/121301
- US-A1- 2007 019 780
- US-A1- 2017 269 174

## Description

The present disclosure relates to a computer-implemented method for providing parameter data for carrying out at least one control scan with a computed tomography scanner arrangement, a system for providing parameter data for carrying out at least one control scan with a computed tomography scanner arrangement and a respective computer program element.

Prior art related to this technical field is disclosed, for example, in WO 2015/121301 A1, US 2017/0269174 A1 and US 2007/0019780 A1.

For some medical procedures, like vertebroplasties, kyphoplasties, biopsies, ablations, drainages or endoleak repairs, it is necessary to bring objects, for example a needle-shaped medical equipment into a patient's body. For such procedures, it is common use, that the examination is supported with repeated online computed tomography imaging in order to control a current position of the object in the patient. Due to the material of these objects, which are typically made of metal, and their geometrical shape, image artifacts can usually not be avoided when the objects are introduced into the patient's body.

It is a common clinical workflow to insert the object, e.g. so-called needles, along paths that are completely within the scan plane of the computed tomography scanner. The reason is that those paths are typically more ergonomic, easier to plan and easier to follow during the medical procedure. But this comes at the drawback of increased image artifacts in the reconstructed control scan images. Typical artifacts are dark streaks along the object path which extend beyond the physical position of the object. Those artifacts are caused by the physical effects of beam-hardening and scatter and the image quality degrading impact of those effects is strongest for the projections which are aligned parallel to the object path. Here, at least for detector pixels that are in the shadow of the object, x-rays are attenuated along the whole object which results in a quite low primary signal, i.e. the signal without artificial contributions, e.g., from scattered radiation, behind the object. In the extreme case, when the dose of the computed tomography scans which are done to control the object position during the medical procedure is reduced to a low level, it may happen that parts of the object disappear in the computed tomography image. The reason for this is that in some of the projection data that are acquired during the computed tomography scan, the measurements that are in the shadow of the object are below the detection level of the x-ray detector and result in a zero-measurement. In these cases, the signal from the object is superimposed, for example by scatter artifacts, quantum noise or electronics noise, so that it cannot be restored in the reconstructed computed tomography image.

In this context, it has become apparent that there is a further need to provide a method and/or a system allowing an improved reconstruction of computed tomography images of a computed tomography scanner arrangement, in particular allowing an improved reconstruction of computed tomography images of control scans of a computed tomography scanner arrangement.

It is therefore an object of the present disclosure to provide a method and/or a system providing an improved reconstruction of computed tomography images of a computed tomography scanner arrangement, in particular providing an improved reconstruction of computed tomography images of control scans of a computed tomography scanner arrangement.

These and other objects, which become apparent upon reading the following description, are solved by the subject matters of the independent claims. The dependent claims refer to preferred embodiments of the present disclosure.

In one aspect of the present disclosure, a computer-implemented method for providing parameter data for carrying out at least one control scan with a computed tomography scanner arrangement is disclosed, comprising:
receiving object path data, at least comprising geometric information about a planned movement path of an object;
receiving predefined control scan data, at least comprising information about the position and orientation of at least one scan plane of at least one planned control scan for controlling the movement path of the object;
receiving an evaluation model configured to provide parameter data for performing the at least one control scan at least depending on the received object path data and the received control scan data;
applying the evaluation model, in particular, to at least the object path data and the predefined control scan data, and providing parameter data for performing the at least one control scan, wherein at least the object path data and the predefined control scan data are input data of the evaluation model.

In other words, the present disclosure proposes to evaluate, by the evaluation model, the object path and, in particular, the predefined position and orientation of the scan plane for the at least one planned control scan, whether there is a risk/probability of image artifacts when the planned control scan is performed. If the evaluation model determines that there is no risk or that the risk is below a predefined threshold value, the already predefined control scan data can be used to perform the control scan without modification by the parameter data. If the evaluation model determines that there is a risk or that the risk is above a specified threshold value, adjustments/modifications to the predefined control scan data may be made by means of the parameter data trying to reduce the risk of image artifacts in the control scan. These adjustments/modifications may relate, for example, to the radiation dose settings and/or the orientation of adjustable moving parts of the computed tomography scanner arrangement adopting the scan plane of the control scan.

Therefore, the present disclosure proposed a method which could evaluated potential vanishing object/needle issues in advance before a control scan is performed. Moreover, if a potential vanishing object/needle issue is evaluated, it is possible to automatically adapt the parameters/settings of a control scans in advance, so that the vanishing object/needle artifact may not show up in a reconstructed computed tomography scanner image.

The term "parameter data" is to be understood broadly in this context and includes, in particular, all data relating to the specific execution of a control scan. For example, parameters for the dosage of X-ray radiation, parameters relating to the positioning and alignment of movable parts of the computed tomography scanner arrangement and the like.

The term "object" is also to be understood broadly in this context and includes all medical devices that can be introduced into a patient and whose position can be checked by computed tomography scanner images. For example, an object can be so-called needles, such as biopsy needles or the like.

The term "object path data" is also to be understood broadly in the present case and may include data that relates to the geometry of the object and a planned movement of the object through a patient. For example, the object path data may include the geometry data of an object and a path that this object is to be moved through a patient's body from a starting point to an end point.

The term "predefined control scan data" is also to be understood broadly in the present case and may include information about the position and orientation of at least one scan plane of at least one planned control scan. The predefined control scan data may also include other predefined parameters, such as predefined X-ray doses, movements and/or trajectories of the computed tomography scanner arrangement during the control scan and the like.

The term "evaluation model" is also to be understood broadly in this context and includes simple fall group distinctions, i.e. predefined parameter data output for certain object path data and predefined control scan data. However, more complex evaluation models may also be used. In an example, the evaluation model may comprise at least one trained algorithm, wherein the term "trained algorithm" or "trained evaluation model" as used herein is to be understood broadly in the present case. The algorithm may be a machine learning algorithm. The algorithm may comprise decision trees, naive bayes classifications, nearest neighbors, neural networks, convolutional or recurrent neural networks, transformers, generative adversarial networks, support vector machines, linear regression, logistic regression, random forest, gradient boosting algorithms and/or a diffusion model. Such an algorithm, in particular machine learning algorithm, is termed "intelligent" because it is capable of being "trained."

The algorithm may be trained using records of training data. A record of training data comprises training input data and corresponding training output data. In the context of the present disclosure, training data may, for example be object path data, predefined control scan data and at least one parameter data output for performing the at least one control scan. The training output data of a record of training data is the result that is expected to be produced by the algorithm when being given the training input data of the same record of training data as input. The deviation between this expected result and the actual result produced by the algorithm is observed and rated by means of a "loss function". This loss function may be used as feedback for adjusting the parameters of the internal processing chain of the algorithm. For example, the parameters may be adjusted with the optimization goal of minimizing the values of the loss function that result when all training input data are fed into the algorithm and the outcome is compared with the corresponding training output data. The result of this training is that given a relatively small number of records of training data as "ground truth", the algorithm is enabled to perform its job well for a number of records of input data that is higher by many orders of magnitude. Notably, the evaluation model may comprise several or even a large number of different evaluation routines/sub-models. It is also possible to use mixed evaluation routines that combine different evaluation approaches.

In an embodiment of the computer-implemented method, the evaluation model may be configured to calculate and evaluate the intersection length of the radiation cone with the planned object path for a projection of the at least one control scan. In this respect, the evaluation model may be configured to provide parameter data based on a comparison of the intersection length and at least one predefined threshold value. For example, if there is a projection where the intersection length of the radiation cone with the planned object/needle path exceeds a certain predefined threshold, for such a projection a certain risk of vanishing object/needle artifacts may be evaluated. If an intersection length matches a needle path, a high risk of vanishing object/needle artifacts may be evaluated

In an embodiment of the computer-implemented method, the method may further comprise: receiving dose data, comprising planned dose settings for the at least one planned control scan; receiving tissue data, comprising tissue information at least in the area of the planned movement path of the object;
wherein the evaluation model is further configured to provide parameter data for performing the at least one control scan further depending on the dose data and the tissue data; and wherein the dose data and the tissue data are further input data of the evaluation model.

By means of further evaluating the planned/predetermined dose settings of the planned control scan and the tissue data/information in the area of the planned control scan, the risk of vanishing object/needle artifacts may be evaluated with higher accuracy. In this respect, at least tissue data may be received relating to the tissue through which the scan passes, so that the damping of the radiation by the tissue may be considered by the evaluation model. The tissue data may be provided, for example, by a previously performed scan, e.g. a so-called planning scan. Using the tissue data of the patient, the total, i.e. by means of tissue and object/needle, attenuation for the vulnerable projections identified in the above-mentioned step may be determined. Based on the control scan dose settings, e.g. tube current and voltage, the signal that the detector would detect in the shadow of the object/needle for the projections may be determined. If this signal is above the detection threshold of the computed tomography scanner arrangement or a predefined detection threshold, it may be assumed that the projections in question may not produce relevant image artifacts. If the signal in the object/needle shadow is below the detection threshold or a predefined detection threshold, it may be assumed that the projections may produce image artifacts, e.g. vanishing object/needle artifacts, and a change in the control scan setup by means of the parameter data may be triggered.

In an embodiment of the computer-implemented method, the parameter data may comprise tilting, alignment and/or positioning information/data with respect to a position, alignment and/or tilt of a gantry arrangement of the computed tomography scanner arrangement. A tilting mechanism of a computed tomography scanner arrangement may be used to tilt the scan plane of the control scan so that the intersection of the object path with the radiation cone may be reduced. Thereby, the gantry's angles of inclination and rotation may be optimized so that the angle between the resulting scan plane and the object/needle path is as close as possible to 90°. Using such a geometry setting for the scan ensures that the cross-section of the object/needle path with the scan plane is as small as possible and that only a fraction of the projections of a full 360° scan are needed to reconstruct and determine a correct position of the object in the reconstructed computed tomography image for small, highly attenuating objects without interesting internal structure.

In an embodiment of the computer-implemented method, the parameter data may comprise dose setting data causing the computed tomography scanner arrangement to use a harder spectrum for the control scan. For example, 150 kV with tin filtration instead of 120 kV. Notably, computed tomography scans typically use a tube current-based dose modulation to optimize patient dose and image quality. When changing the tube voltage for the entire scan, the tube current profile may be configured in such a way that the total dose to the patient may remain constant.

In an embodiment of the computer-implemented method, the parameter data may comprise dose setting data causing the computed tomography scanner arrangement to modify a tube current modulation profile increasing the tube current for at least one projection of the object/needle path, in particular for those projections for which a risk of image artifacts was identified. In particular, by means of changing the tube current modulation profile, the tube current may be increased for projections that are parallel or within a predefined threshold to the object/needle path. The total patient dose may be kept constant by reducing the tube current for the remaining projections that are considered to have no or a low risk for image artifacts, e.g. which are not parallel to the object/needle path. Alternatively, the total patient dose may also be increased, which can be justified by the improved image quality of the control scans.

In an embodiment of the computer-implemented method, the parameter data may comprise dose setting data causing the computed tomography scanner arrangement to modify a tube voltage using a harder spectrum for at least one projection of the object/needle path, in particular for those projections for which a risk of image artifacts was identified. In particular, by means of changing the tube voltage, a harder spectrum may be used for projections that are parallel or within a predefined threshold to the object/needle path. For example, the spectrum may be modified to 150 kV with tin filtration instead of 120 kV.

In an embodiment of the computer-implemented method, the parameter data may comprise: tilting, alignment and/or positioning information/data with respect to a position, alignment and/or tilt of a gantry arrangement of the computed tomography scanner arrangement;
dose setting data causing the computed tomography scanner arrangement to use a higher/harder tube voltage for the control scan;
dose setting data causing the computed tomography scanner arrangement to modify a tube current modulation profile increasing the tube current for at least one projection of the object/needle path, in particular for those projections for which a risk of image artifacts was identified; and/or
dose setting data causing the computed tomography scanner arrangement to modify a tube voltage using a harder spectrum for at least one projection of the object/needle path, in particular for those projections for which a risk of image artifacts was identified.

In an embodiment of the computer-implemented method, the parameter data may comprise scan data causing the computed tomography scanner arrangement to turn on the tube only for predetermined projections and/or to turn on only tubes according to a predetermined schedule. Today, standard spiral or sequence scans are usually used for computed tomography guided procedures to perform the control scans for object/needle positioning. In order to reduce the overall dose to a patient, these images are typically acquired at a reduced dose, but still a full set of raw data is acquired. Full here means that a standard computed tomography scanner image may be reconstructed from the acquired raw data, i.e. the raw data set contains at least projections within an angular interval of 180° + fan angle of the computed tomography scanner. In this respect, the total dose can be reduced, for example, by turning on the tube only for a fraction of the predetermined projections, depending on the geometry of the planned needle path.

In case a so called static computed tomography scanner arrangement with distributed radiation sources is used, the overall radiation dose may be further reduced. Static computed tomography scanner arrangement means that the scanner is not assembled according to the geometry principle of the third generation. A static computed tomography scanner arrangement has a ring detector, e.g. a full 360° detector ring, and a source ring, wherein the source ring comprises several small X-ray tubes or a distributed source, e.g. based on field-effect emitter cathodes, with a constant increment. During data collection, these tubes can be switched on and off independently and, in any order, allowing that for a control scan only predetermined X-ray tubes or distributed radiation sources are turned on corresponding to the needle/object path.

In an embodiment of the computer-implemented method, the method may further comprise: providing control data for controlling a computed tomography scanner arrangement based on the parameter data for the at least one control scan.

By providing control data based on the parameter data, the computed tomography scanner arrangement may be fully automated to adapt to the planned control scan.

A further aspect of the present disclosure relates to a system for providing parameter data for carrying out at least one control scan with a computed tomography scanner arrangement, comprising:
a processing circuitry;
a storage medium; and
a data interface;
wherein the storage medium comprises a computer program that comprises instructions which when the program is executed, cause the processing circuitry to carry out the above-explained computer-implemented method;
wherein the data interface is configured to receive input data for the evaluation model including the object path data and the predefined control scan data.

In an embodiment of the system, the system may comprise a computed tomography scanner, preferably comprising a tilting/movement mechanism configured to change the gantry's angle of inclination and rotation.

In an embodiment of the system, the system may comprise a computed tomography scanner with distributed radiation sources. A static computed tomography scanner arrangement has a ring detector, e.g. a full 360° detector ring, and a radiation source ring, wherein the radiation source ring comprises several small X-ray tubes or a distributed radiation source, e.g. based on field-effect emitter cathodes, with a constant increment.

A further aspect of the present disclosure relates to a computer program element with instructions, which, when executed on computing devices of a computing environment, is configured to carry out the steps of the above-described computer-implemented, in particular, in an above-described system. The computer program element might be stored on a computing unit of a computing device, which might also be part of an embodiment. This computing unit may be configured to perform or induce performing of the steps of the method described above. Moreover, it may be configured to operate the components of the above-described system. The computing unit can be configured to operate automatically and/or to execute the orders of a user. The computing unit may include a data processor. A computer program may be loaded into a working memory of a data processor. The data processor may thus be equipped to carry out the method according to one of the preceding embodiments. This exemplary embodiment of the present disclosure covers both, a computer program that right from the beginning uses the present disclosure and computer program that by means of an update turns an existing program into a program that uses the present disclosure. Moreover, the computer program element might be able to provide all necessary steps to fulfill the procedure of an exemplary embodiment of the method as described above.

According to a further exemplary embodiment of the present disclosure, a computer readable medium, such as a CD-ROM, USB stick, a downloadable executable or the like, is presented wherein the computer readable medium has a computer program element stored on it, which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present disclosure, a medium for making a computer program element available for downloading is provided, which computer program element is arranged to perform a method according to one of the previously described embodiments of the present disclosure.

A further aspect of the present disclosure relates to a use of
object path data, at least comprising geometric information about a planned movement path of an object;
predefined control scan data, at least comprising information about the position and orientation of at least one scan plane of at least one planned control scan for controlling the movement path of the object; and/or
an evaluation model configured to provide parameter data for performing the at least one control scan at least depending on the received object path data and the received control scan data;
in an above-described computer-implemented method for providing parameter data for carrying out at least one control scan with a computed tomography scanner arrangement.

This and embodiments described herein relate to the methods, the systems, the apparatuses, the computer program element, the computer-readable storage medium, the use lined out above and vice versa. Advantageously, the benefits provided by any of the embodiments and examples equally apply to all other embodiments and examples and vice versa.

The term "control data" as used herein is to be understood broadly in the present case and comprises any data structure/information structure that is suitable for controlling a robot component, for example in digital form and/or in analog form.

The term "data" as used herein is to be understood broadly in the present case and represents any kind of data. Data may be single numbers/numerical values, a plurality of a numbers/numerical values, a plurality of a numbers/numerical values being arranged within a list, 2 dimensional maps or 3 dimensional maps, but are not limited thereto.

The term "providing" and the term "receiving" used herein are to be understood broadly in the present case and could be related to any of providing, receiving, querying, measuring, calculating, determining, transmitting of data, but are not limited thereto. Data may be provided to a user via a user interface, in particular, by depicting/showing the data on a display. Data may be provided to another device by transmitting the data to the other device. Data may be received from another device. The data may by queried from the other device, measured by the other device, calculated by the other device, determined by the other device and/or transmitted by the other device.

In the following particularly preferred embodiments are disclosed, which may be combined with the above-disclosed methods, systems, apparatuses, devices and/or use cases. The embodiments described herein may be combined unless specifically noted otherwise. Features and advantages described with respect to one aspect of the disclosure may be applied to other aspects of the disclosure.

In the following, the present disclosure is further described with reference to the enclosed figures:
- Figure 1: illustrates a flow diagram of a computer-implemented method for providing parameter data for carrying out at least one control scan with a computed tomography scanner arrangement according to an embodiment of the present disclosure;
- Figure 2: illustrates a system for providing parameter data for carrying out at least one control scan with a computed tomography scanner arrangement according to an embodiment of the present disclosure;
- Figure 3: is a schematic side view of a computed tomography scanner arrangement according to an embodiment of the present disclosure; and
- Figure 4: is a schematic top view of a computed tomography scanner arrangement according to an embodiment of the present disclosure.

The following embodiments are mere examples for implementing the method, the system, disclosed herein and shall not be considered limiting.

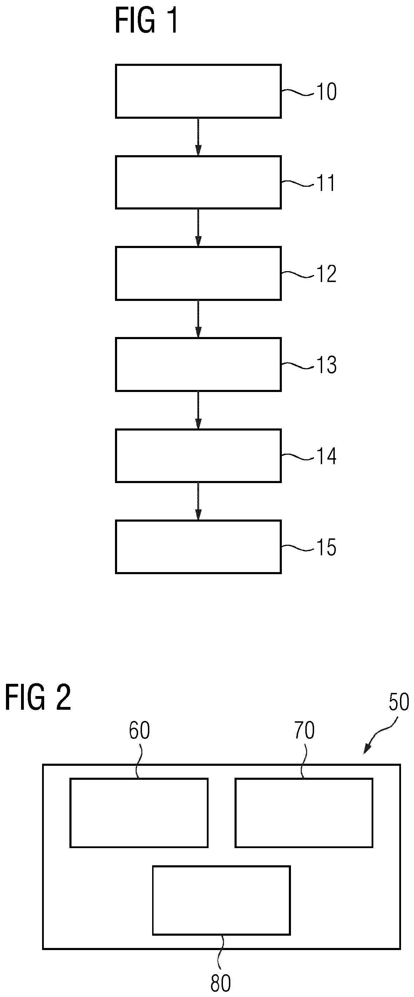

Figure 1 illustrates a flow diagram of a computer-implemented method for providing parameter data for carrying out at least one control scan with a computed tomography scanner arrangement according to an embodiment of the present disclosure.

In a first step 10, object/needle path data, at least comprising geometric information about a planned movement path of an object are received. Such object/needle path data may be provided by a previously performed scan, e.g. a so-called planning scan. For example, the object path data may include the geometry data of an object and a path that this object is to be moved through a patient's body from a starting point to an end point.

In a second step 11, predefined control scan data, at least comprising information about the position and orientation of at least one scan plane of at least one planned control scan for controlling the movement path of the object are received. The predefined control scan data may be entered in a previous step automatically, partially automatically and/or manually. For example, the predefined control scan data may be predefined by default program settings of a computed tomography scanner arrangement.

In a further step 12, an evaluation model configured to provide parameter data for performing the at least one control scan at least depending on the received object path data and the received control scan data is received. The evaluation model may comprise predefined parameter data output for certain object path data and predefined control scan data. However, more complex evaluation models may also be used. For example, the evaluation model may comprise at least one trained algorithm, wherein the term "trained algorithm" or "trained evaluation model". In an example of the present disclosure, the evaluation model is configured to calculate and evaluate the intersection length of the radiation cone with the planned object path for a projection of the at least one control scan. In this respect, the evaluation model may be configured to provide parameter data based on a comparison of the intersection length and at least one predefined threshold value. For example, if there is a projection where the intersection length of the radiation cone with the planned object/needle path exceeds a certain predefined threshold, for such a projection a certain risk of vanishing object/needle artifacts may be evaluated. If an intersection length matches a needle path, a high risk of vanishing object/needle artifacts may be evaluated.

In a further step 13, the evaluation model is applied and the parameter data for performing the at least one control scan is provided, wherein at least the object path data and the predefined control scan data are input data of the evaluation model.

In an example of the present disclosure, the parameter data may comprise:
tilting, alignment and/or positioning information/data with respect to a position, alignment and/or tilt of a gantry arrangement of the computed tomography scanner arrangement;
dose setting data causing the computed tomography scanner arrangement to use a higher/harder tube voltage for the control scan;
dose setting data causing the computed tomography scanner arrangement to modify a tube current modulation profile increasing the tube current for at least one projection of the object/needle path, in particular for those projections for which a risk of image artifacts was identified; and/or
dose setting data causing the computed tomography scanner arrangement to modify a tube voltage using a harder spectrum for at least one projection of the object/needle path, in particular for those projections for which a risk of image artifacts was identified.

Thus, the present disclosure proposes to evaluate, by the evaluation model, the object path and, in particular, the predefined position and orientation of the scan plane for the at least one planned control scan, whether there is a risk/probability of image artifacts when the planned control scan is performed. If the evaluation model determines that there is no risk or that the risk is below a predefined threshold value, the already predefined control scan data can be used to perform the control scan without modification by the parameter data. If the evaluation model determines that there is a risk or that the risk is above a specified threshold value, adjustments/modifications to the predefined control scan data may be made by means of the parameter data trying to reduce the risk of image artifacts in the control scan. These adjustments/modifications may relate, for example, to the radiation dose settings and/or the orientation of adjustable moving parts of the computed tomography scanner arrangement adopting the scan plane of the control scan.

In an optional further step 14, dose data comprising planned dose settings for the at least one planned control scan are received. In a further optional step 15, tissue data comprising tissue information at least in the area of the planned movement path of the object are received. Such tissue data of a patient may be provided by a previously performed scan, e.g. a so-called planning scan.

Figure 2 illustrates a system 50 for providing parameter data for carrying out at least one control scan with a computed tomography scanner arrangement, comprising: a processing circuitry 60, a storage medium 70, and a data interface 80. The storage medium 70 comprises a computer program with instructions which when the program is executed, cause the processing circuitry 60 to carry out the computer-implemented method for providing parameter data for carrying out at least one control scan with a computed tomography scanner arrangement according to an embodiment of the present disclosure. The data interface 80 is configured to receive at least input data for the evaluation model including the object path data and the predefined control scan data.

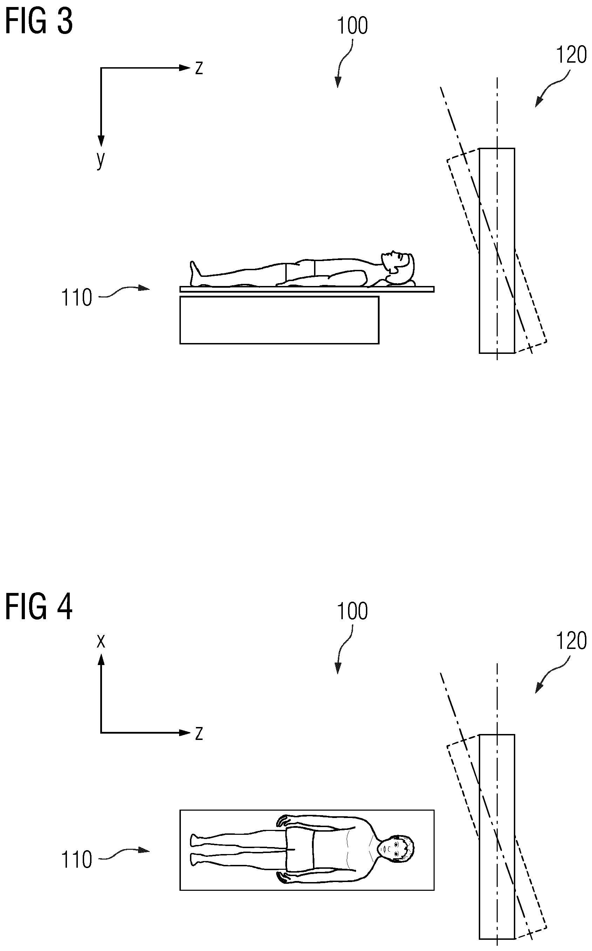

In Figures 3 and 4, an example of a computed tomography scanner arrangement 100 is illustrated. The computed tomography scanner arrangement 100 comprises a patient table 110, on which a patient may be placed, and a gantry 120 comprising the radiation source(s) and the detector means. The computed tomography scanner arrangement 100 may comprise a tilting/rotating mechanism which may be used to tilt and/or rotate the gantry and thus the scan plane of the control scan so that the intersection of the object path with the radiation cone may be reduced. Thereby, the gantry's angle of inclination (cf. Figure 3) relative to the patient table 110 around a horizontal tilt axis, the horizontal tilt axis being perpendicular to a horizontal longitudinal axis of the patient table 110, and/or the gantry's angle of rotation (cf. Figure 4) relative to the patient table 110 around a vertical rotation axis may be optimized, for example, so that the angle between the resulting scan plane and the object/needle path is as close as possible to 90°. Using such a geometry setting for the scan may ensure that the cross-section of the object/needle path with the scan plane is as small as possible and that only a fraction of the projections of a full 360° scan are needed to reconstruct and determine a correct position of the object in the reconstructed computed tomography image for small, highly attenuating objects without interesting internal structure.

As indicated in Figures 3 and 4, usually, the spatial coding is based on an X-Y-Z coordinate system. The Z-axis is usually defined as an axis of symmetry of the gantry of the computed tomography scanner arrangement through an opening of the gantry. In the usual setup of a computed tomography scanner arrangement, the Z-axis is oriented horizontally and runs centrally through the opening of the gantry. As indicated in the Figures 3 and 4, the patient is usually brought into the opening parallel to the Z-axis on the patient table 110. Together with the Z-coordinate axis, an X-coordinate axis and a Y-coordinate axis span a space, wherein the coordinate axes are preferably orthogonal to one another and the X-coordinate axis is horizontal and the Y-coordinate axis is vertical.

The present disclosure has been described in conjunction with a preferred embodiment as examples as well. However, other variations can be understood and effected by those persons skilled in the art and practicing the claimed invention, from the studies of the drawings, this disclosure and the claims. Notably, in particular, the any steps presented can be performed in any order, i.e. the present invention is not limited to a specific order of these steps. Moreover, it is also not required that the different steps are performed at a certain place or at one node of a distributed system, i.e. each of the steps may be performed at a different node using different equipment/data processing units.

As used herein "determining" also includes "estimating, calculating, initiating or causing to determine", "generating" also includes "initiating or causing to generate" and "providing" also includes "initiating or causing to determine, generate, select, send, query or receive".

In the claims as well as in the description the word "comprising" does not exclude other elements or steps and the indefinite article "a" or "an" does not exclude a plurality. A single element or other unit may fulfill the functions of several entities or items recited in the claims.

The mere fact that certain measures are recited in the mutual different dependent claims does not indicate that a combination of these measures cannot be used in an advantageous implementation. Independent of the grammatical term usage, individuals with male, female or other gender identities are included within the term.

## Claims

1. Computer-implemented method for providing parameter data for carrying out at least one control scan with a computed tomography scanner arrangement (100), comprising:
receiving (10) object path data, at least comprising geometric information about a planned movement path of an object;
receiving (11) predefined control scan data, at least comprising information about the position and orientation of at least one scan plane of at least one planned control scan for controlling the movement path of the object;
receiving (12) an evaluation model configured to provide parameter data for performing the at least one control scan at least depending on the received object path data and the received control scan data;
applying (13) the evaluation model and providing the parameter data for performing the at least one control scan, wherein at least the object path data and the predefined control scan data are input data of the evaluation model.

2. Computer-implemented method according to claim 1, wherein the evaluation model is configured to calculate and evaluate the intersection length of a radiation cone with the planned object path for a projection of the at least one control scan.

3. Computer-implemented method according to claim 2, wherein the evaluation model is configured to provide parameter data based on a comparison of the intersection length and at least one predefined threshold value.

4. Computer-implemented method according to any one of the preceding claims, the method further comprising:
receiving dose data (14), comprising planned dose settings for the at least one planned control scan;
receiving tissue data (15), comprising tissue information at least in the area of the planned movement path of the object;
wherein the evaluation model is further configured to provide parameter data for performing the at least one control scan further depending on the dose data and the tissue data; and wherein the dose data and the tissue data are further input data of the evaluation model.

5. Computer-implemented method according to any one of the preceding claims, the parameter data may comprise tilting, alignment and/or positioning information/data with respect to a position, alignment and/or tilt of a gantry arrangement of the computed tomography scanner arrangement.

6. Computer-implemented method according to any one of the preceding claims, wherein, the parameter data comprise dose setting data causing the computed tomography scanner arrangement to use a higher tube voltage for the control scan.

7. Computer-implemented method according to any one of the preceding claims, wherein the parameter data comprise dose setting data causing the computed tomography scanner arrangement to modify a tube current modulation profile increasing the tube current for at least one projection of the object/needle path.

8. Computer-implemented method according to any one of the preceding claims, wherein the parameter data comprise scan data causing the computed tomography scanner arrangement to turn the tube on only for predetermined projections and/or to turn on only tubes according to a predetermined schedule.

9. Computer-implemented method according to any one of the preceding claims, further comprising:
providing control data for controlling a computed tomography scanner arrangement based on the parameter data for the at least one control scan.

10. System (50) for providing parameter data for carrying out at least one control scan with a computed tomography scanner, comprising:
a processing circuitry (60);
a storage medium (70); and
a data interface (80);
wherein the storage medium (70) comprises a computer program that comprises instructions which when the program is executed, cause the processing circuitry (60) to carry out the method according to any one of the claims 1 to 9;
wherein the data interface (80) is configured to receive input data for the evaluation model including the object path data and the predefined control scan data.

11. System (50) according to claim 10, wherein the system comprises a computed tomography scanner comprising a tilting/movement mechanism configured to change the gantry's angle of inclination and/or rotation.

12. System (50) according to claim 10 or 11, wherein the system comprises a computed tomography scanner comprising a ring detector and a radiation source ring comprising several X-ray tubes or a distributed radiation source.

13. Computer program element with instructions, which, when executed on computing devices of a computing environment, is configured to carry out the steps of the computer-implemented method according to any one of the claims 1 to 9, in particular in a system according to any one of the claims 10 to 12.

14. Use of
object path data, at least comprising geometric information about a planned movement path of an object;
predefined control scan data, at least comprising information about the position and orientation of at least one scan plane of at least one planned control scan for controlling the movement path of the object; and/or
an evaluation model configured to provide parameter data for performing the at least one control scan at least depending on the received object path data and the received control scan data;
in a computer-implemented method according to any one of claims 1 to 9.

## Patentansprüche

1. Computerimplementiertes Verfahren zum Bereitstellen von Parameterdaten zum Durchführen wenigstens eines Überprüfungsscans mit einer Computertomographie-Scanneranordnung (100), umfassend:
Empfangen (10) von Objektwegdaten, die wenigstens geometrische Informationen über einen geplanten Bewegungsweg eines Objekts umfassen;
Empfangen (11) vorgegebener Überprüfungsscandaten, die wenigstens Informationen über die Position und Orientierung wenigstens einer Scanebene wenigstens eines geplanten Überprüfungsscans zum Überprüfen des Bewegungswegs des Objekts umfassen;
Empfangen (12) eines Bewertungsmodells, das dazu ausgelegt ist, Parameterdaten zum Durchführen des wenigstens einen Überprüfungsscans wenigstens abhängig von den empfangenen Objektwegdaten und den empfangenen Überprüfungsscandaten bereitzustellen;
Anwenden (13) des Bewertungsmodells und Bereitstellen der Parameterdaten zum Durchführen des wenigstens einen Überprüfungsscans, wobei wenigstens die Objektwegdaten und die vorgegebenen Überprüfungsscandaten Eingabedaten des Bewertungsmodells sind.

2. Computerimplementiertes Verfahren nach Anspruch 1, wobei das Auswertungsmodell dazu ausgelegt ist, die Schnittlänge eines Strahlungskegels mit dem geplanten Objektweg für eine Projektion des wenigstens einen Überprüfungsscans zu berechnen und auszuwerten.

3. Computerimplementiertes Verfahren nach Anspruch 2, wobei das Bewertungsmodell dazu ausgelegt ist, Parameterdaten auf der Grundlage eines Vergleichs der Schnittlänge und wenigstens eines vorgegebenen Schwellenwerts bereitzustellen.

4. Computerimplementiertes Verfahren nach einem der vorstehenden Ansprüche, wobei das Verfahren ferner umfasst:
Empfangen von Dosisdaten (14), die geplante Dosiseinstellungen für den wenigstens einen geplanten Überprüfungsscan umfassen;
Empfangen von Gewebedaten (15), die Gewebeinformationen wenigstens in dem Bereich des geplanten Bewegungswegs des Objekts umfassen;
wobei das Bewertungsmodell ferner dazu ausgelegt ist, Parameterdaten zum Durchführen des wenigstens einen Überprüfungsscans ferner abhängig von den Dosisdaten und den Gewebedaten bereitzustellen; und wobei die Dosisdaten und die Gewebedaten ferner Eingabedaten des Bewertungsmodells sind.

5. Computerimplementiertes Verfahren nach einem der vorstehenden Ansprüche, wobei die Parameterdaten Neigungs-, Ausrichtungs- und/oder Positionierungsinformationen/daten in Bezug auf eine Position, Ausrichtung und/oder Neigung einer Gantry-Anordnung der Computertomographie-Scanneranordnung umfassen können.

6. Computerimplementiertes Verfahren nach einem der vorstehenden Ansprüche, wobei die Parameterdaten Dosiseinstellungsdaten umfassen, die bewirken, dass die Computertomographie-Scanneranordnung eine höhere Röhrenspannung für den Überprüfungsscan verwendet.

7. Computerimplementiertes Verfahren nach einem der vorstehenden Ansprüche, wobei die Parameterdaten Dosiseinstellungsdaten umfassen, die bewirken, dass die Computertomographie-Scanneranordnung ein Röhrenstrom-Modulationsprofil modifiziert, das den Röhrenstrom für wenigstens eine Projektion des Objekt-/Nadelwegs erhöht.

8. Computerimplementiertes Verfahren nach einem der vorstehenden Ansprüche, wobei die Parameterdaten Scandaten umfassen, die bewirken, dass die Computertomographie-Scanneranordnung die Röhre nur für vorgegebene Projektionen einschaltet und/oder nur Röhren gemäß einem vorgegebenen Zeitplan einschaltet.

9. Computerimplementiertes Verfahren nach einem der vorstehenden Ansprüche, ferner umfassend:
Bereitstellen von Steuerdaten zum Steuern einer Computertomographie-Scanneranordnung auf der Grundlage der Parameterdaten für den wenigstens einen Überprüfungsscan.

10. System (50) zum Bereitstellen von Parameterdaten zum Durchführen wenigstens eines Überprüfungsscans mit einem Computertomographiescanner, umfassend:
eine Verarbeitungsschaltung (60);
ein Speichermedium (70); und
eine Datenschnittstelle (80);
wobei das Speichermedium (70) ein Computerprogramm umfasst, das Anweisungen umfasst, die bei Ausführung des Programms die Verarbeitungsschaltung (60) veranlassen, das Verfahren nach einem der Ansprüche 1 bis 9 auszuführen;
wobei die Datenschnittstelle (80) dazu ausgelegt ist, Eingabedaten für das Bewertungsmodell einschließlich der Objektwegdaten und der vorgegebenen Überprüfungsscandaten zu empfangen.

11. System (50) nach Anspruch 10, wobei das System einen Computertomographiescanner umfasst, der einen Neigungs-/Bewegungsmechanismus umfasst, der dazu ausgelegt ist, den Neigungswinkel und/oder die Drehung der Gantry zu ändern.

12. System (50) nach Anspruch 10 oder 11, wobei das System einen Computertomographiescanner umfasst, der einen Ringdetektor und einen Strahlungsquellenring umfasst, der mehrere Röntgenröhren oder eine verteilte Strahlungsquelle umfasst.

13. Computerprogrammelement mit Anweisungen, die dazu ausgelegt sind, bei Ausführung auf Rechenvorrichtungen einer Rechenumgebung Schritte des computerimplementierten Verfahrens nach einem der Ansprüche 1 bis 9, insbesondere in einem System nach einem der Ansprüche 10 bis 12, durchzuführen.

14. Verwendung von
Objektwegdaten, die wenigstens geometrische Informationen über einen geplanten Bewegungsweg eines Objekts umfassen;
vorgegebenen Überprüfungsscandaten, die wenigstens Informationen über die Position und Orientierung wenigstens einer Scanebene wenigstens eines geplanten Überprüfungsscans zum Überprüfen des Bewegungswegs des Objekts umfassen; und/oder
eines Bewertungsmodells, das dazu ausgelegt ist, Parameterdaten zum Durchführen des wenigstens einen Überprüfungsscans wenigstens abhängig von den empfangenen Objektwegdaten und den empfangenen Überprüfungsscandaten bereitzustellen;
bei einem computerimplementierten Verfahren nach einem der Ansprüche 1 bis 9.

## Revendications

1. Procédé mis en œuvre par ordinateur pour donner une donnée de paramètre pour effectuer au moins un balayage de vérification par un agencement (100) de scanner de tomodensitométrie informatisée, comprenant :
recevoir (10) une donnée de chemin d'un objet, comprenant au moins une information géométrique sur un chemin planifié de déplacement d'un objet ;
recevoir (11) une donnée de balayage de vérification définie à l'avance, comprenant au moins une information sur la position et l'orientation d'au moins un plan de balayage d'au moins un balayage de vérification planifié pour vérifier le chemin de déplacement de l'objet ;
recevoir (12) un modèle d'évaluation configuré pour donner une donnée de paramètre pour effectuer le au moins un balayage de vérification en fonction de la donnée reçue du chemin de l'objet et de la donnée reçue de balayage de vérification ;
appliquer (13) le modèle d'évaluation et donner la donnée de paramètre pour effectuer le au moins un balayage de vérification, dans lequel au moins la donnée de chemin de l'objet et la donnée définie à l'avance de balayage de vérification sont des données d'entrée du modèle d'évaluation.

2. Procédé mis en œuvre par ordinateur suivant la revendication 1, dans lequel le modèle d'évaluation est configuré pour calculer et évaluer la longueur d'intersection d'un cône de rayonnement avec le chemin planifié de l'objet pour une projection du au moins un balayage de vérification.

3. Procédé mis en œuvre par ordinateur suivant la revendication 2, dans lequel le modèle d'évaluation est configuré pour donner une donnée de paramètre sur la base d'une comparaison de la longueur d'intersection et d'au moins une valeur de seuil définie à l'avance.

4. Procédé mis en œuvre par ordinateur suivant l'une quelconque des revendications précédentes, le procédé comprenant en outre :
recevoir une donnée (14) de dose, comprenant des réglages planifiés de dose pour le au moins un balayage planifié de vérification ;
recevoir une donnée (15) de tissu, comprenant une information de tissu au moins dans la zone du chemin planifié de déplacement de l'objet ;
dans lequel le modèle d'évaluation est configuré en outre pour donner une donnée de paramètre pour effectuer le au moins un balayage de vérification en fonction en outre de la donnée de dose et de la donnée de tissu ; et dans lequel la donnée de dose et la donnée de tissu sont d'autres données d'entrée du modèle d'évaluation.

5. Procédé mis en œuvre par ordinateur suivant l'une quelconque des revendications précédentes, dans lequel la donnée de paramètre peut comprendre une information / une donnée d'inclinaison, d'alignement et/ou de mise en position par rapport à une position, un alignement et/ou une inclinaison d'un agencement de portique de l'agencement de scanner de tomodensitométrie informatisée.

6. Procédé mis en œuvre par ordinateur suivant l'une quelconque des revendications précédentes, dans lequel la donnée de paramètre comprend une donnée de réglage de dose faisant que l'agencement de scanner de tomodensitométrie informatisée utilise une tension de tube assez élevée pour le balayage de vérification.

7. Procédé mis en œuvre par ordinateur suivant l'une quelconque des revendications précédentes, dans lequel la donnée de paramètre comprend une donnée de réglage de dose faisant que l'agencement de scanner de tomodensitométrie informatisée modifie un profil de modulation de courant de tube augmentant le courant de tube pendant au moins une projection du chemin de l'objet / aiguille.

8. Procédé mis en œuvre par ordinateur suivant l'une quelconque des revendications précédentes, dans lequel la donnée de paramètre comprend une donnée de balayage faisant que l'agencement de scanner de tomodensitométrie informatisée met en circuit le tube seulement pendant des projections déterminées à l'avance et/ou met en circuit seulement des tubes suivant un plan déterminé à l'avance.

9. Procédé mis en œuvre par ordinateur suivant l'une quelconque des revendications précédentes, comprenant en outre :
donner la donnée de commande pour commander un agencement de scanner de tomodensitométrie informatisée sur la base de la donnée de paramètre pour le au moins un balayage de vérification.

10. Système (50) pour donner une donnée de paramètre pour effectuer au moins un balayage de vérification par un scanner de tomodensitométrie informatisée, comprenant :
un circuit (60) de traitement ;
un support (70) de mémoire ; et
une interface (80) de donnée ;
dans lequel le support (70) de mémoire comprend un programme d'ordinateur, qui comprend des instructions qui, lorsque le programme est exécuté, font que le circuit (60) de traitement effectue le procédé suivant l'une quelconque des revendications 1 à 9 ;
dans lequel l'interface (80) de donnée est configurée pour recevoir une donnée d'entrée pour le modèle d'évaluation incluant la donnée du chemin de l'objet et la donnée de balayage de vérification définie à l'avance.

11. Système (50) suivant la revendication 10, dans lequel le système comprend un scanner de tomodensitométrie informatisée comprenant un mécanisme d'inclinaison et/ou de déplacement configuré pour changer l'angle d'inclinaison et/ou de rotation du portique.

12. Système (50) suivant la revendication 10 ou 11, dans lequel le système comprend un scanner de tomodensitométrie informatisée comprenant un détecteur annulaire et un anneau de source de rayonnement réparti.

13. Elément de programme d'ordinateur avec des instructions qui, lorsqu'elles sont exécutées sur des dispositifs informatiques d'un environnement informatique, est configuré pour effectuer les stades du procédé mis en œuvre par ordinateur suivant l'une quelconque des revendications 1 à 9, en particulier dans un système suivant l'une quelconque des revendications 10 à 12.

14. Utilisation de
une donnée de chemin d'un objet, comprenant au moins une information géométrique sur un chemin planifié de déplacement d'un objet ;
une donnée définie à l'avance de balayage de vérification comprenant au moins une information sur la position et l'orientation d'au moins un plan de balayage d'au moins un balayage planifié de vérification pour vérifier le chemin de déplacement de l'objet ; et/ou
un modèle d'évaluation configuré pour donner une donnée de paramètre pour effectuer le au moins un balayage de vérification en fonction au moins de la donnée reçue de chemin de l'objet et de la donnée reçue de balayage de vérification ;
dans un procédé mis en œuvre par ordinateur suivant l'une quelconque des revendications 1 à 9.
